# FASCICULE DE BREVET EUROPEEN

(11) **EP 0 826 669 B1**
(45) Date de publication et mention de la délivrance du brevet: **30.06.1999**
(21) Numéro de dépôt: 97401912.7
(22) Date de dépôt: 08.08.1997
(51) Int. Cl.: C07D 209/40, A61K 7/13

(54) **Compositions de teinture des fibres kératiniques contenant des dérivés 2-iminoindoliniques**
Mittel zum Färben von Keratinfasern beinhaltend 2-Iminoindol Derivaten
Composition for dyeing keratin fibres containing 2-iminoindol derivatives

(30) Priorité: 23.08.1996 FR 9610413
(43) Date de publication de la demande: 04.03.1998
(73) Titulaire: L'OREAL, 75008 Paris (FR)
(72) Inventeur: Terranova, Eric, 92600 Asnieres (FR); Fadli, Aziz, 93150 Le Blanc-Mesnil (FR); Lagrange, Alain, 77700 Coupvray (FR)
(74) Mandataire: Goulard, Sophie

(56) Documents cités:
- EP-A- 0 428 441
- WO-A-92/17157
- FR-A- 2 008 797
- : "", TETRAHEDRON 1971, , , Vol. 27, no. , pages 775 à 787
- : "", J.ORG.CHEM. , 1973, , , Vol. 38, no. 1, pages 11 à 16
- : "", TETRAHEDRON 1971, , 00-00-1971, Vol. 27, no. , pages 775 à 787
- : "", J.ORG.CHEM., , 00-00-1973, Vol. 38, no. 1, pages 11 à 16

## Description

L'invention a pour objet une composition pour la teinture d'oxydation des fibres kératiniques contenant au moins un dérivé 2-imino indolinique à titre de coupleur et au moins une base d'oxydation.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques (WO 92/17157).

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est-à-dire permettre d'obtenir des écarts de coloration les plus faibles possible tout au long d'une même fibre kératinique, qui peut être en effet différemment sensibilisée (i.e. abîmée) entre sa pointe et sa racine.

Or, la demanderesse vient maintenant de découvrir qu'il est possible d'obtenir de nouvelles teintures puissantes, peu sélectives et particulièrement résistantes, capables d'engendrer des colorations intenses dans des nuances variées, en utilisant des dérivés 2-imino indoliniques particuliers. Ces composés (pour partie nouveaux en soi) sont de plus aisément synthétisables.

Cette découverte est à la base de la présente invention.

L'invention a donc pour objet une composition pour la teinture des fibres kératiniques et en particulier des fibres kératiniques humaines telles que les cheveux, caractérisée par le fait qu'elle comprend, dans un milieu approprié pour la teinture :
- à titre de coupleur, au moins un dérivé 2-iminoindolinique de formule (I), et/ou au moins l'un de ses sels d'addition avec un acide, et/ou une de leurs possibles formes tautomères : 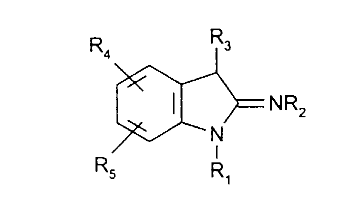 dans laquelle:
- R₁ représente un atome d'hydrogène, un radical alkyle en C₁-C₄, monohydroxyalkyle en C₁-C₄, polyhydroxyalkyle en C₂-C₄ un alcoxy(C₁-C₄)carbonyle ou un radical acyle en C₂-C₄ ;
- R₂ représente un atome d'hydrogène, un radical alkyle en C₁-C₄, phényle ou arylsulfonyle ;
- R₃ représente un atome d'hydrogène, un radical alkyle en C₁-C₄ pouvant être substitué par un ou plusieurs groupements hydroxy, alcoxy en C₁-C₄, amino, monoalkyl(C₁-C₄)amino, dialkyl(C₁-C₄)amino, cyano ou aryle ;
- R₄ et R₅, identiques ou différents, désignent un atome d'hydrogène, un radical hydroxyle, alkyle en C₁-C₄, alcoxy en C₁-C₄, amino, monoalkyl(C₁-C₄)amino, dialkyl(C₁-C₄)amino, aralkyle en C₇-C₁₁, un atome d'halogène ou un groupe nitro ;
- et au moins une base d'oxydation.

Dans la formule (I) ci-dessus, les groupements alkyle en C₁-C₄ et alcoxy en C₁-C₄ peuvent être linéaires ou ramifiés et parmi les atomes d'halogène, on peut citer le chlore, le brome, l'iode et le fluor.

Dans la formule (I) ci-dessus, les groupements aryle peuvent désigner par exemple phényle, thiophène, furanne.

La formule (I) telle que définie ci-dessus peut donner lieu aux deux formes tautomères suivantes :

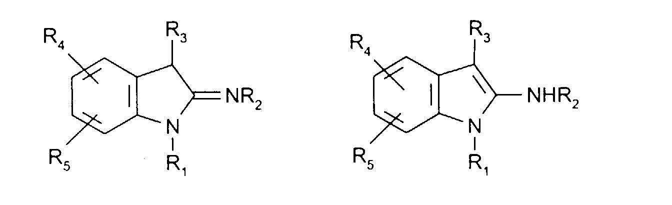

La prépondérance et/ou la stabilité de chacune de ces deux formes tautomères dépendra de la nature des substituants R₁, R₂ et R₃.

Les colorations obtenues avec la composition de teinture conforme à l'invention, sont de nuances variées, puissantes, peu sélectives et présentent d'excellentes propriétés de résistance à la fois vis-à-vis des agents atmosphériques tels que la lumière et les intempéries et vis-à-vis de la transpiration et des différents traitements que peuvent subir les cheveux (shampooings, déformations permanentes).

Parmi les dérivés 2-imino indoliniques de formule (I), utilisables à titre de coupleurs dans les compositions conformes à l'invention, on peut notamment citer :
- le 4-benzyloxy-1,3-dihydro-indol-2-ylidèneamine,
- le 4-méthoxy-1,3-dihydro-indol-2-ylidèneamine,
- le 2-imino-2,3-dihydro-1 H-indol-4-ol,
- le 5-chloro-7-méthoxy-1,3-dihydro-indol-2-ylidèneamine,
- le 5,6-diméthoxy-1,3-dihydro-indol-2-ylidèneamine,
- le 5,6-dihydroxy-1,3-dihydro-indol-2-ylidèneamine,
- le 1,3-dihydro-indol-2-ylidèneamine,
ainsi que leurs sels d'addition d'acide.

Le ou les dérivés 2-imino indoliniques de formule (I) représentent de préférence de 0,0005 à 12 % en poids environ du poids total de la composition tinctoriale, et encore plus préférentiellement de 0,005 à 6 % en poids environ de ce poids.

La nature de la ou des bases d'oxydation pouvant être utilisées dans la composition tinctoriale selon l'invention n'est pas critique. Cette ou ces bases d'oxydation sont de préférence choisies parmi les paraphénylènediamines, les bis-phénylalkylènediamines, les paraaminophénols, les ortho-aminophénols, les bases hétérocycliques, et leurs sels d'addition avec un acide.

Parmi les paraphénylènediamines utilisables à titre de bases d'oxydation dans la composition tinctoriale selon l'invention, on peut notamment citer les composés répondant à la formule (Il) suivante, et leurs sels d'addition avec un acide : 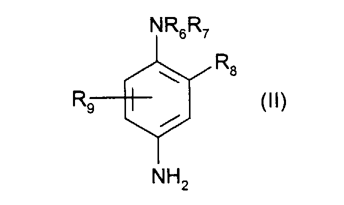 dans laquelle :
R₆ représente un atome d'hydrogène, un radical alkyle en C₁-C₄, monohydroxyalkyle en C₁-C₄, polyhydroxyalkyle en C₂-C₄ ou alcoxy(C₁-C₄)alkyle(C₁-C₄),
R₇ représente un atome d'hydrogène, un radical alkyle en C₁-C₄, monohydroxyalkyle en C₁-C₄ ou polyhydroxyalkyle en C₂-C₄,
R₈ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄,
R₉ représente un atome d'hydrogène ou un radical alkyle en C₁-C₄.

Dans la formule (Il) ci-dessus, et lorsque R₈ est différent d'un atome d'hydrogène, alors R₆ et R₇ représentent de préférence un atome d'hydrogène et R₈ est de préférence identique à R₉, et lorsque R₈ représente un atome d'halogène, alors R₆, R₇ et R₉ représentent de préférence un atome d'hydrogène.

Parmi les paraphénylènediamines de formule (Il) ci-dessus, on peut plus particulièrement citer la paraphénylènediamine, la paratoluylènediamine, la 2-isopropyl paraphénylène-diamine, la 2-β-hydroxyéthyl paraphénylènediamine, la 2-β-hydroxyéthyloxy para-phénylènediamine, la 2,6-diméthyl paraphénylènediamine, la 2,6-diéthyl paraphénylène-diamine, la 2,3-diméthyl paraphénylènediamine, la N,N-bis-(β-hydroxyéthyl) para-phénylènediamine, le 4-amino 1-(β-méthoxyéthyl) amino benzène, la 2-chloro para-phénylènediamine, et leurs sels d'addition avec un acide.

Parmi les bis-phénylalkylènediamines utilisables à titre de bases d'oxydation dans la composition tinctoriale selon l'invention, on peut notamment citer les composés répondant à la formule (III) suivante, et leurs sels d'addition avec un acide : 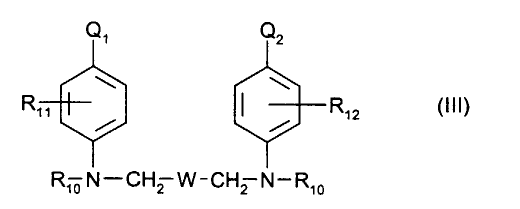 dans laquelle :
Q₁ et Q₂, identiques ou différents, représentent un radical hydroxyle ou NHR₁₃ dans lequel R₁₃ représente un atome d'hydrogène ou un radical alkyle en C₁-C₄,
R₁₀ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
R₁₁ et R₁₂, identiques ou différents, représentent un atome d'hydrogène ou d'halogène ou un radical alkyle en C₁-C₄,
W représente un radical pris dans le groupe constitué par les radicaux suivants : 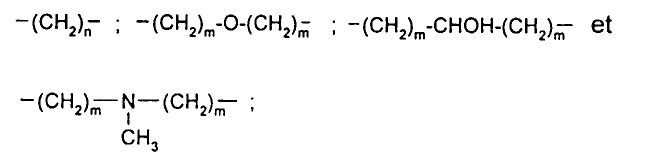 dans lesquels n est un nombre entier compris entre 0 et 8 inclusivement et m est un nombre entier compris entre 0 et 4 inclusivement.

Parmi les bis-phénylalkylènediamines de formule (III) ci-dessus, on peut plus particulièrement citer le N,N'-bis-(β-hydroxyéthyl) N,N'-bis-(4'-aminophényl) 1,3-diamino 2-propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (III), le N,N'-bis-(β-hydroxyéthyl) N,N'-bis-(4'-aminophényl) 1,3-diamino propanol ou l'un de ses sels d'addition avec un acide sont particulièrement préférés.

Parmi les para-aminophénols utilisables à titre de bases d'oxydation dans la composition tinctoriale selon l'invention, on peut notamment citer les composés répondant à la formule (IV) suivante, et leurs sels d'addition avec un acide : 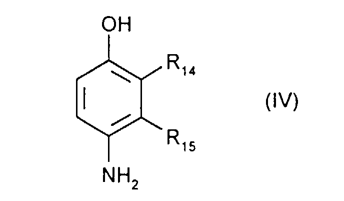 dans laquelle :
R₁₄ représente un atome d'hydrogène, un radical alkyle en C₁-C₄, monohydroxyalkyle en C₁-C₄, alcoxy(C₁-C₄)alkyle(C₁-C₄) ou aminoalkyle en C₁-C₄,
R₁₅ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₄ ou R₁₅ représente un atome d'hydrogène.

Parmi les paraaminophénols de formule (IV) ci-dessus, on peut plus particulièrement citer le paraaminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols utilisables à titre de bases d'oxydation dans la composition tinctoriale selon l'invention, on peut notamment citer le 2-amino phénol, le 2-amino 1-hydroxy 5-méthyl benzène, le 2-amino 1-hydroxy 6-méthyl benzène, le 5-acétamido 2-amino phénol, et leurs sels d'addition avec un acide.

Parmi les bases hétérocycliques utilisables à titre de bases d'oxydation dans la composition tinctoriale selon l'invention, on peut notamment citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-333 495, comme la 2,4,5,6-tétra-aminopyrimidine, la 4-hydroxy 2,5,6-triamino-pyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969 et WO 94/08970 comme le 4,5-diamino 1-méthyl pyrazole, le 3,4-diamino pyrazole, et leurs sels d'addition avec un acide.

Selon l'invention, la ou les bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale selon l'invention peut également renfermer un ou plusieurs coupleurs additionnels différents des dérivés 2-imino indoliniques de formule (I) et/ou un ou plusieurs colorants directs de façon à faire varier ou enrichir en reflets les nuances obtenues avec les bases d'oxydation.

Les coupleurs additionnels utilisables dans la composition selon l'invention peuvent être choisis parmi les coupleurs utilisés de façon classique en teinture d'oxydation et parmi lesquels on peut notamment citer les métaphénylènediamines, les métaaminophénols, les métadiphénols et les coupleurs hétérocycliques tels que par exemple les dérivés indoliques, les dérivés indoliniques, et leurs sels d'addition avec un acide.

Ces coupleurs peuvent notamment être choisis parmi le 2-méthyl 5-amino phénol, le 5-N-(β-hydroxyéthyl)amino 2-méthyl phénol, le 3-amino phénol, le 1,3-dihydroxybenzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxyindole, le 4-hydroxy N-méthyl indole, la 6-hydroxy indoline, et leurs sels d'addition avec un acide.

Lorsqu'ils sont présents, ces coupleurs additionnels représentent de préférence de 0,0005 à 5 % en poids environ du poids total de la composition tinctoriale, et encore plus préférentiellement de 0,005 à 3 % en poids environ de ce poids.

Les sels d'addition avec un acide de la ou des bases d'oxydation et/ou des coupleurs additionnels utilisables dans la composition tinctoriale de l'invention sont notamment choisis parmi les chlorhydrates, les bromhydrates, les sulfates et les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol, les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 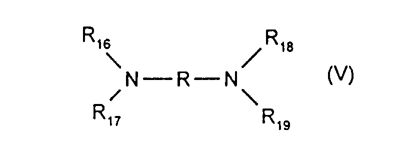 dans laquelle R est un reste propylène éventuellement substitué par un groupement hydroxyle ou un radical alkyle en C₁-C₄ ; R₁₆, R₁₇, R₁₈ et R₁₉, identiques ou différents, représentent un atome d'hydrogène, un radical alkyle en C₁-C₄ ou hydroxyalkyle en C₁-C₄.

La composition tinctoriale selon l'invention peut également renfermer divers adjuvants utilisés classiquement dans les compositions pour la teinture des cheveux, tels que des agents tensioactifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet l'utilisation des dérivés 2-imino indoliniques de formule (I) ci-dessus, à titre de coupleur, en association avec au moins une base d'oxydation pour la teinture d'oxydation des fibres kératiniques et particulier des fibres kératiniques humaines telles que les cheveux.

Un autre objet de l'invention est un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les composés de formule (I), utilisés à titre de coupleurs dans le cadre de la présente invention, sont soit des composés connus, soit pour certains des composés nouveaux en soi.

Les composés de formule (lA) particuliers correspondant à la formule (I) dans laquelle R₁ et R₂ sont des atomes d'hydrogène, peuvent être obtenus selon un procédé de préparation, décrit dans les documents RG Glushkov USSR Patent 179 320 (1965) et Chem. Abstr 65 , 2225 (1966), et répondant au schéma A suivant :

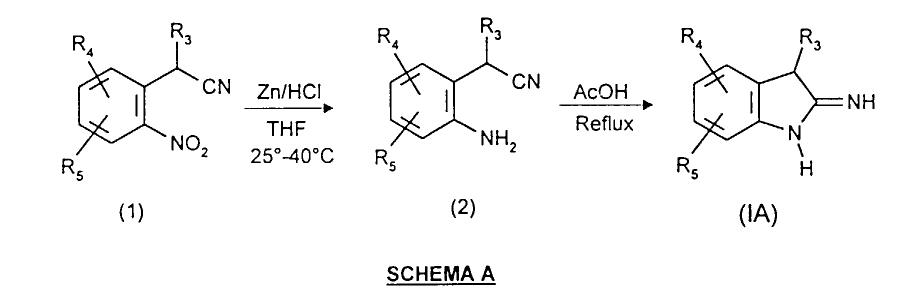

Dans le schéma A défini ci-dessus, les significations des radicaux R₃, R₄ et R₅ dans les formules (1) et (2) sont identiques à celles indiquées précédemment dans la formule (I).

Il s'agit d'un procédé en deux étapes à partir de composés de départ de formule (1) ayant pour structure celle d'ortho-nitro phényl acétonitriles dont la méthode de préparation est connue dans la littérature (M. MAKOSZA, J. WINIARSKI, Acc. Chem. Res., 87, 1987, 282 ; M. MAKOSZA, W. DANIKIEWICZ, K. WOJCIECHOWSKI, Liebigs, Ann. Chem., 1988, 203).

La première étape est soit une réduction chimique en présence d'un solvant organique à l'aide de métaux comme le zinc ou l'étain, soit une hydrogénation sélective à l'aide d'un catalyseur comme le palladium ou le platine. Elle conduit à des dérivés de structure ortho-amino phényl acétonitrile (formule(2)). On utilise plus particulièrement le zinc en poudre en milieu acide chlorhydrique pour cette réaction. Les solvants utilisés sont de préférence des éthers et plus particulièrement le tétrahydrofuranne (THF). La température de réaction est comprise de préférence entre 25°C et la température de reflux du solvant et plus particulièrement entre 25 et 40°C.

La seconde étape est une réaction de cyclisation en milieu acide en présence d'un solvant organique. On utilise plus particulièrement l'acide acétique. La température de réaction est celle de reflux du solvant. Le produit final de formule (I') est isolé de préférence sous forme de sel d'addition avec un acide de préférence sous forme de chlorhydrate. On l'obtient par précipitation du milieu réactionnel en milieu acide, par exemple, pour obtenir un chlorhydrate, on fait passer un courant de HCI gazeux en fin de réaction.

Les composés de formule (IB) particuliers correspondant à la formule (I) dans laquelle R₁ est un atome d'hydrogène et R₂ est différent d'un atome d'hydrogène et les composés de formule (IC) particuliers correspondant à la formule (I) dans laquelle R₁ et R₂ sont tous les deux différents d'un atome d'hydrogène, peuvent être obtenus selon un procédé de préparation, faisant référence à la littérature, et répondant au schéma B suivant :

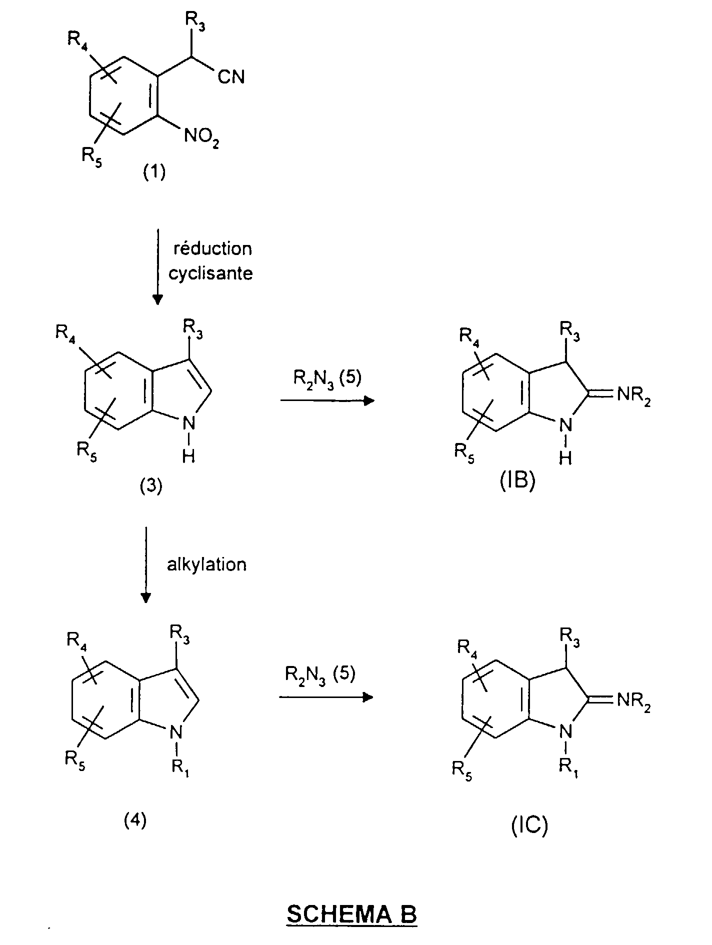

Le composé (1) peut être traité dans des conditions de réduction cyclisante selon des méthodes connues, comme par exemple celle décrite par MAKOSZA M. & coll. dans Liebigs Ann. Chem., 203, (1988) pour conduire à l'indole (3).
L'indole (3) peut être alkylé pour conduire à l'indole (4) selon des méthodes classiques décrites dans *"Heterocyclic compounds : Indoles"* part Il p. 72-73, édité par N.J. HOULIAN, Wiley-interscience.

Les composés (3) et (4) peuvent réagir avec un azide de structure (5) pour conduire respectivement aux 2-iminoindolines (IB) et (IC) selon une méthode déjà décrite [HARMON R.E. & coll., J. Org. Chem. 38(1), 11, (1973)].

Les composés de structures (IB) et (IC) peuvent également être obtenus par réaction d'une amine R₂NH₂ respectivement sur un dérivé 2-indolinethione de structure (6) et (7) comme décrit par HINO T. & coll. dans Tetrahedron 27, 775, (1971) et représenté dans le schéma C suivant :

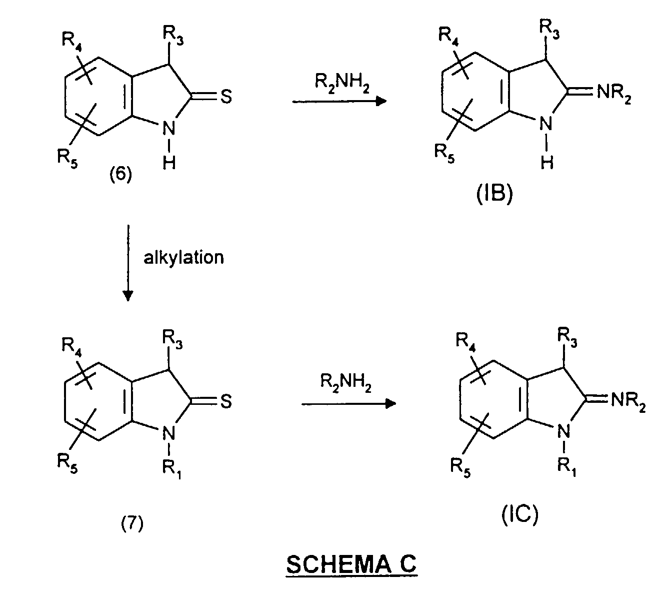

Les exemples qui suivent sont destinés à illustrer l'invention sans pour autant en limiter la portée.

### EXEMPLES DE PREPARATION

### EXEMPLE n° 1 : Préparation du chlorhydrate de 1,3-Dihydro-indol-2-ylidèneamine

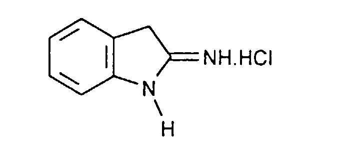

Dans un ballon tricol de 50 ml équipé d'un thermomètre et d'un réfrigérant, on a solubilisé 2 g de (2-nitro-phényl)-acétonitrile dans un mélange THF/HCI à 37% soit 10 ml/10 ml.
On a introduit 5 grammes de zinc par petites portions à une température inférieure à 40° C. Après complète addition, on a maintenu l'agitation pendant 15 minutes puis on a filtré l'excès de zinc. Le filtrat a été évaporé à sec. On a repris le chlorhydrate de (2-amino-phényl)-acétonitrile au reflux dans 25 ml d'acide acétique. Après une heure de chauffage, on a saturé la solution d'acide chlorhydrique gazeux. On a filtré le précipité. On l'a séché sous vide sur anhydride phosphorique et sur potasse. On a obtenu 1 g de chlorhydrate de 1,3-dihydro-indol-2-ylidèneamine (rendement = 48%).
RMN ¹H et ¹³C conformes à la structure.

| RMN ¹H (DMSO d6 ; 400 mhz) | | |
|---|---|---|
| **δ (ppm)** | **multiplicité** | **intégration** |
| 4.19 | s | 2 |
| 7.13 | ddd | 1 |
| 7.22 | d | 1 |
| 7.30 | ddd | 1 |
| 7.41 | d | 1 |
| 9.95-10.21 | 2 s élargis | 2 |
| 12.42 | s élargi | 1 |

### EXEMPLE n° 2 : Préparation du chlorhydrate de 4-méthoxy-1,3-dihydro-indol-2-ylidèneamine

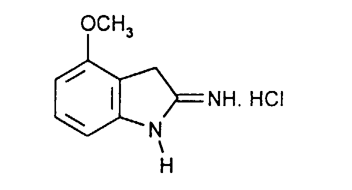

### 1ère étape: Préparation du chlorhydrate de (2-amino-6-méthoxy-phényl)-acétonitrile

Dans un ballon tricol de 100 ml équipé d'un thermomètre et d'un réfrigérant, on a solubilisé 3,84 g de (2-méthoxy-6-nitro-phényl)-acétonitrile dans un mélange THF/HCI = 1/1 (40 ml).
On a introduit 7,8 g de zinc par petites portions à une température inférieure à 40°C. Après complète addition, on a maintenu l'agitation pendant 15 minutes puis on a filtré l'excès de zinc. Le filtrat a été évaporé à sec. On a récupéré 3 g de chlorhydrate de (2-amino-6-méthoxy-phényl)-acétonitrile (Rendement = 75%).

### 2ème étape: Préparation du chlorhydrate de 4-méthoxy-1,3-dihydro-indol-2-ylidèneamine

Dans un ballon tricol de 100 ml équipé d'un thermomètre et d'un réfrigérant, on a porté au reflux 3 g du chlorhydrate de (2-amino-6-méthoxy-phényl)-acétonitrile dans 25 ml d'acide acétique pendant une heure. Après évaporation de l'acide sous vide, on a récupéré 2,9 g de chlorhydrate de 4-méthoxy-1,3-dihydro-indol-2-ylidèneamine après séchage sous vide sur anhydride phosphorique et potasse (Rendement = 96%).
RMN ¹H et ¹³C conformes à la structure.

| RMN ¹H (DMSO d6; 400 mhz) | | |
|---|---|---|
| **δ (ppm)** | **multiplicité** | **intégration** |
| 3.84 | s | 3 |
| 4.06 | s | 2 |
| 6.80-6.83 | 2 d | 2 |
| 7.31 | t | 1 |
| 9.72-9.98 | 2s | 2 |
| 12.09 | s | 1 |

### EXEMPLE n° 3 : Préparation du bromhydrate de 2-imino-2,3-dihydro-1H-indol-4-ol

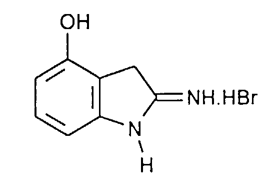

Dans un ballon tricol de 25 ml équipé d'un thermomètre et d'un réfrigérant, on a porté au reflux pendant quatre heures 2 g de chlorhydrate 4-méthoxy-1,3-dihydro-indol-2-ylidèneamine dans 8 ml d'acide bromhydrique à 60%. On a laissé revenir le milieu à température ambiante. Le bromhydrate de 2-imino-2,3-dihydro-1H-indol-4-ol a cristallisé. On l'a filtré et on l'a lavé à l'éther de pétrole. On a récupéré 1,6 g de produit après séchage sous vide sur anhydride phosphorique et potasse (Rendement = 70%).
RMN ¹H et ¹³C conformes à la structure.

| RMN ¹H (DMSO d6; 400 mhz) | | |
|---|---|---|
| **δ (ppm)** | **multiplicité** | **intégration** |
| 4.02 | s | 2 |
| 6.60-6.67 | 2 d | 2 |
| 7.13 | t | 1 |
| 9.64-9.95 | 2s + s élargi | 3 |
| 11.98 | s | 1 |

### EXEMPLE n°4 : Préparation du chlorhydrate de 4-benzyloxy-1,3-dihydro-indol-2-ylidèneamine.

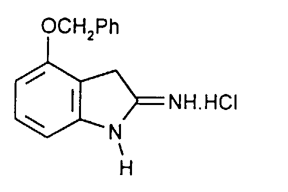

### 1ère étape : Préparation du (2-amino-6-benzyloxy-phényl)-acétonitrile

Dans un réacteur SVL de 2 litres équipé d'un réfrigérant et d'un thermomètre, on a solubilisé 135 g de (2-benzyloxy-6-nitro-phényl)-acétonitrile dans 1 litre de THF et 0,5 litre d'acide chlorhydrique à 37%.
On a introduit 150 g de zinc par petites portions à une température inférieure à 40° C. Après complète addition, on a maintenu l'agitation pendant 15 minutes puis on a filtré l'excès de zinc.
Le filtrat a été versé sur 10 volumes d'eau glacée. Le précipité a été filtré, lavé à l'eau et à l'éther de pétrole. On a récupéré 80 g de (2-amino-6-benzyloxy-phényl)-acétonitrile après séchage sous vide et sur anhydride phosphorique (Rendement = 67%).

### 2ème étape : Préparation du chlorhydrate de 4-benzyloxy-1,3-dihydro-indol-2-ylidèneamine

Dans un ballon tricol de 500 ml équipé d'un thermomètre et d'un réfrigérant, on a porté au reflux 71,5 g de (2-amino-6-benzyloxy-phényl)-acétonitrile dans 350 ml d'acide acétique pendant une heure. On a laissé revenir le milieu à la température ambiante, puis on l'a saturé avec de l'acide chlorhydrique gazeux. On a filtré le précipité. On l'a lavé à l'éther de pétrole. On a récupéré 63 g de chlorhydrate de 4-benzyloxy-1,3-dihydro-indol-2-ylidèneamine après séchage sous vide sur anhydride phosphorique et potasse (Rendement = 76 %).
RMN ¹H et ¹³C conformes à la structure.

| RMN ¹H (DMSO d6; 400 mhz) | | |
|---|---|---|
| δ **(ppm)** | **multiplicité** | **intégration** |
| 4.10 | s | 2 |
| 5.20 | s | 2 |
| 6.87 | d | 2 |
| 7.26 | t | 1 |
| 7.34 | m | 1 |
| 7.39 | m | 2 |
| 7.46 | m | 2 |
| 10.04-10.38 | 2s | 2 |
| 12.51 | s | 1 |

### EXEMPLE n° 5 : Préparation du chlorhydrate de 5-chloro-7-méthoxy-1,3-dihydro-indol-2-ylidèneamine

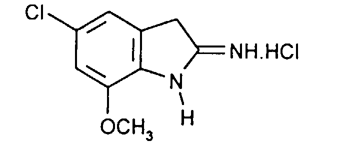

### 1ère étape : Préparation du (2-amino-5-chloro-3-méthoxy-phényl)-acétonitrile

Conformément à l'exemple n° 3, on a réduit 22,7 g de (5-chloro-3-méthoxy-2-nitro-phényl)-acétonitrile dissous dans un mélange THF/HCI à 37% = 100 ml / 70 ml avec 26 g de zinc en poudre. On a obtenu 19 g de (2-amino-5-chloro-3-méthoxy-phényl)-acétonitrile (Rendement = 97%).

### 2ème étape : Préparation du chlorhydrate de 5-chloro-7-méthoxy-1,3-dihydro-indol-2-ylidèneamine

Conformément à l'exemple n° 3, on a porté au reflux 15 g de (2-amino-5-chloro-3-méthoxy-phényl)-acétonitrile dans 150 ml d'acide acétique. On a obtenu 11,2 g de chlorhydrate de 5-chloro-7-méthoxy-1,3-dihydro-indol-2-ylidèneamine (Rendement = 63,6 %).
RMN ¹H et ¹³C conformes à la structure.

| RMN ¹H (DMSO d6; 400 mhz) | | |
|---|---|---|
| δ **(ppm)** | **multiplicité** | **intégration** |
| 3.91 | s | 3 |
| 4.23 | s | 2 |
| 7.13 | d | 1 |
| 7.16 | d | 1 |
| 9.13-10.05 | 2s | 2 |
| 12.51 | s | 1 |

### EXEMPLE n° 6 : Préparation du chlorhydrate de 5,6-diméthoxy-1,3-dihydro-indol-2-ylidèneamine

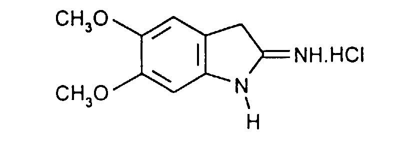

Dans un ballon tricol de 250 ml équipé d'un thermomètre et d'un réfrigérant, on a solubilisé 22,2 g de (4,5-diméthoxy-2-nitro-phényl)-acétonitrile dans un mélange THF/HCI à 37 % = 150 ml / 60 ml.
On a introduit 26 g de zinc en poudre par petites portions à une température inférieure à 40°C. L'addition était exothermique. Après complète addition, on a maintenu l'agitation pendant 15 minutes puis on a filtré l'excès de zinc. On a saturé le filtrat avec de l'HCl gazeux, puis on a filtré le précipité obtenu. On l'a lavé à l'éther de pétrole, puis on l'a séché sous vide sur anhydride phosphorique et potasse. On a obtenu 15 g de chlorhydrate de 5,6-diméthoxy-1,3-dihydro-indol-2-ylidèneamine (Rendement = 65 %).
RMN ¹H et ¹³C conformes à la structure.

| RMN ¹H (DMSO d6; 400 mhz) | | |
|---|---|---|
| δ **(ppm)** | **multiplicité** | **intégration** |
| 3.73 | s | 3 |
| 3.76 | s | 3 |
| 4.09 | s | 2 |
| 6.83 | s | 1 |
| 7.11 | s | 1 |
| 9.49-9.78 | 2 s | 2 |
| 11.73 | s | 1 |

### EXEMPLE n° 7 : Préparation du chlorhydrate de 5,6-dihydroxy-1,3-dihydro-indol-2-ylidèneamine

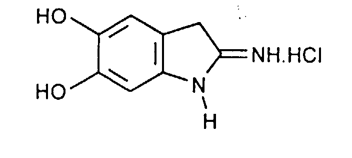

Conformément à l'exemple n°6, on a solubilisé 1,9 g de (4,5-dihydroxy-2-nitro-phényl)-acétonitrile dans un mélange THF/HCI à 37 % = 20 ml / 20 ml qu'on a traité avec 5 g de zinc. Après traitement, on a obtenu 1.8 g de chlorhydrate de 5,6-dihydroxy-1,3-dihydro-indol-2-ylidèneamine (Rendement = 90 %).
RMN ¹H et ¹³C conformes à la structure.

| RMN ¹H (DMSO d6; 400 mhz) | | |
|---|---|---|
| **δ (ppm)** | **multiplicité** | **intégration** |
| 3.97 | s | 2 |
| 6.67 | s | 1 |
| 6.80 | s | 1 |
| 8.00-9.00 | m | 2 |
| 9.42-9.66 | 2 s | 2 |
| 11.69 | s | 1 |

### EXEMPLES DE FORMULATION

### EXEMPLES DE FORMULATION 1 et 2 :

On a préparé les compositions tinctoriales suivantes (teneurs en grammes) :

| **Compositions** | **1** | **2** |
|---|---|---|
| 2-imino-2,3-dihydro-1H-indol-4-ol, HBr | 1,14 | |
| 5,6-dihydroxy-1,3-dihydro-indol-2-ylidèneamine, HCl | | 0,68 |
| Paraphénylènediamine | 0,54 | |
| Paratoluylènediamine | | 0,66 |
| Support de teinture commun | (**) | (**) |
| Eau déminéralisée q.s.p. | 100 g | 100 g |

| | | |
|---|---|---|
| (**) : support de teinture commun : - Alcool oléique polyglycérolé à 2 moles de glycérol 4,0 g - Alcool oléique polyglycérolé à 4 moles de glycérol à 78 % de matières actives (M.A.) 5,69 g M.A. - Acide oléique 3,0 g - Amine oléique à 2 moles d'oxyde d'éthylène vendue sous la dénomination commerciale ETHOMEEN O12 par la société AKZO 7,0 g - Laurylamino succinamate de diéthylaminopropyle, sel de sodium à 55 % de M.A. 3,0 g M.A. - Alcool oléique 5,0 g - Diéthanolamide d'acide oléique 12,0 g - Propylèneglycol 3,5 g - Alcool éthylique 7,0 g - Dipropylèneglycol 0,5 g - Monométhyléther de propylèneglycol 9,0 g - Métabisulfite de sodium à en solution aqueuse à 35 % de M.A. 0,455 g M.A. - Acétate d'ammonium 0,8 g - Antioxydant, séquestrant q.s. - Parfum, conservateur q.s. - Ammoniaque à 20 % de NH₃ 10,0 g | | |

Au moment de l'emploi, chaque composition tinctoriale 1 et 2 a été mélangée avec un poids égal d'eau oxygénée à 20 volumes (6% en poids).

Chaque mélange obtenu a été appliqué pendant 30 minutes, sur des mèches de cheveux gris naturels à 90 % de blancs, permanentés ou non, à raison de 28 g pour 3 g de cheveux. Après rinçage, lavage avec un shampooing standard et séchage, les mèches ont été teintes dans les nuances figurant dans le tableau I ci-dessous :

**Tableau I**

| **Exemple** | **1** | **2** |
|---|---|---|
| **Nuance obtenue sur cheveux gris naturels à 90% de blancs** | Blond foncé acajou irisé | Blond clair cendré beige |
| **Nuance obtenue sur cheveux gris à 90% de blancs permanentés** | Châtain clair cuivré acajou | Blond foncé naturel cendré doré |

## Revendications

1. Composition pour la teinture des fibres kératiniques et en particulier des fibres kératiniques humaines telles que les cheveux, caractérisée par le fait qu'elle comprend, dans un milieu approprié pour la teinture :
- à titre de coupleur, au moins un dérivé 2-iminoindolinique de formule (I), et/ou au moins l'un de ses sels d'addition avec un acide, et/ou au moins une de leurs possibles formes tautomères : 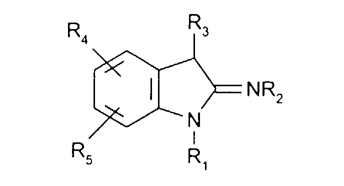 dans laquelle :
- R₁ représente un atome d'hydrogène, un radical alkyle en C₁-C₄, monohydroxyalkyle en C₁-C₄, polyhydroxyalkyle en C₂-C₄ un alcoxy(C₁-C₄)carbonyle ou un radical acyle en C₂-C₄ ;
- R₂ représente un atome d'hydrogène, un radical alkyle en C₁-C₄, phényle ou arylsulfonyle ;
- R₃ représente un atome d'hydrogène, un radical alkyle en C₁-C₄ pouvant être substitué par un ou plusieurs groupements hydroxy, alcoxy en C₁-C₄, amino, monoalkyl(C₁-C₄)amino, dialkyl(C₁-C₄)amino, cyano ou aryle ;
- R₄ et R₅, identiques ou différents, désignent un atome d'hydrogène, un radical hydroxyle, alkyle en C₁-C₄, alcoxy en C₁-C₄, amino, monoalkyl(C₁-C₄)amino, dialkyl(C₁-C₄)amino, aralkyle en C₇-C₁₁, un atome d'halogène ou un groupe nitro ;
- et au moins une base d'oxydation.

2. Composition selon la revendication 1, caractérisée par le fait que les composés de formule (I) sont choisis parmi :
- le 4-benzyloxy-1,3-dihydro-indol-2-ylidèneamine,
- le 4-méthoxy-1,3-dihydro-indol-2-ylidèneamine,
- le 2-imino-2,3-dihydro-1H-indol-4-ol,
- le 5-chloro-7-méthoxy-1,3-dihydro-indol-2-ylidèneamine,
- le 5,6-diméthoxy-1,3-dihydro-indol-2-ylidèneamine,
- le 5,6-dihydroxy-1,3-dihydro-indol-2-ylidèneamine,
- le 1,3-dihydro-indol-2-ylidèneamine,
et leurs sels d'addition d'acide.

3. Composition selon l'une quelconque des revendications précédentes, caractérisée par le fait que les sels d'addition avec un acide des composés de formule (I) sont choisis parmi les chlorhydrates, les bromhydrates, les sulfates, les tartrates et les acétates.

4. Composition selon l'une quelconque des revendications précédentes, caractérisée par le fait que le ou les composés de formule (I) représentent de 0,0005 à 12 % en poids du poids total de la composition tinctoriale.

5. Composition selon la revendication 4, caractérisée par le fait que le ou composés de formule (I) représentent de 0,005 à 6 % en poids du poids total de la composition tinctoriale.

6. Composition selon l'une quelconque des revendications précédentes, caractérisée par le fait que la ou les bases d'oxydation sont choisies parmi les paraphénylènediamines, les bis-phénylalkylènediamines, les para-aminophénols, les ortho-aminophénols, les bases hétérocycliques, et leurs sels d'addition avec un acide.

7. Composition selon l'une quelconque des revendications précédentes, caractérisée par le fait que la ou les bases d'oxydation représentent de 0,0005 à 12 % en poids environ du poids total de la composition tinctoriale.

8. Composition selon la revendication 7, caractérisée par le fait que la ou les bases d'oxydation représentent de 0,005 à 6 % en poids environ du poids total de la composition tinctoriale.

9. Composition selon l'une quelconque des revendications précédentes, caractérisée par le fait qu'elle renferme en outre un ou plusieurs coupleurs additionnels différents des composés de formule (I) et/ou un ou plusieurs colorants directs.

10. Composition selon l'une quelconque des revendications précédentes, caractérisée par le fait que le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

11. Composition selon l'une quelconque des revendications précédentes, caractérisée par le fait qu'elle présente un pH compris entre 3 et 12.

12. Composition selon l'une quelconque des revendications précédentes, caractérisée par le fait qu'elle se présente sous forme de liquides, de crèmes, de gels, ou sous toute autre forme appropriée pour réaliser une teinture des fibres kératiniques, et notamment des cheveux humains.

13. Utilisation des composés de formule (I) telle que définis à l'une quelconque des revendications 1 à 3, à titre de coupleurs dans des compositions pour la teinture d'oxydation des fibres kératiniques et en particulier des fibres kératiniques humaines telles que les cheveux, en association avec au moins une base d'oxydation.

14. Procédé de teinture d'oxydation des fibres kératiniques et en particulier des fibres kératiniques humaines telles que les cheveux, caractérisé par le fait qu'on applique sur ces fibres au moins une composition tinctoriale telle que définie à l'une quelconque des revendications 1 à 12, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

15. Procédé selon la revendication 14, caractérisé par le fait que l'agent oxydant est choisi parmi le peroxyde d'hydrogène, le peroxyde d'urée, les bromates de métaux alcalins, les persels tels que les perborates et persulfates.

16. Dispositif à plusieurs compartiments, ou "kit" de teinture à plusieurs compartiments, dont un premier compartiment renferme une composition tinctoriale telle que définie à l'une quelconque des revendications 1 à 12 et un second compartiment renferme une composition oxydante.

## Patentansprüche

1. Zusammensetzung zum Färben von Keratinfasern und insbesondere menschlichen Keratinfasern, wie dem Haar, dadurch gekennzeichnet, daß sie in einem zum Färben geeigneten Medium enthält:
- als Kuppler mindestens ein 2-Iminoindolinderivat der Formel (I) und/oder mindestens ein Additionssalz dieser Verbindungen mit einer Säure und/oder mindestens eine ihrer möglichen tautomeren Formen: 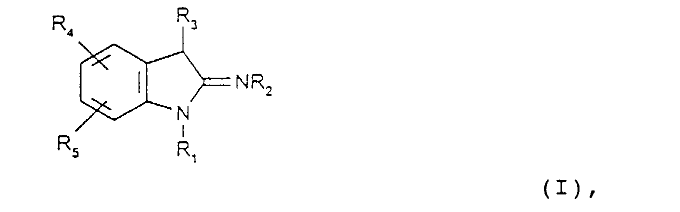 worin bedeuten:
- R₁ Wasserstoff, C₁₋₄-Alkyl, C₁₋₄,-Monohydroxyalkyl, C₂₋₄-Polyhydroxyalkyl, C₁₋₄-Alkoxy-carbonyl oder C₂₋₄-Acyl;
- R₂ Wasserstoff, C₁₋₄-Alkyl, Phenyl oder Arylsulfonyl;
- R₃ Wasserstoff, C₁₋₄-Alkyl, das mit einer oder mehreren Hydroxygruppen substituiert sein kann, C₁₋₄-Alkoxy, Amino, C₁₋₄-Monoalkyl-amino, C₁₋₄-Dialkyl-amino, Cyano oder Aryl;
- R₄ und R₅, die identisch oder voneinander verschieden sind, Wasserstoff, Hydroxy, C₁₋₄-Alkyl, C₁₋₄-Alkoxy, Amino, C₁₋₄-Monoalkyl-amino, C₁₋₄-Dialkyl-amino, C₇₋₁₁-Aralkyl, Halogen oder Nitro;
- und mindestens eine Oxidationsbase.

2. Zusammensetzung nach Anspruch 1, dadurch gekennzeichnet, daß die Verbindungen der Formel (I) ausgewählt sind unter:
- 4-Benzyloxy-1,3-dihydro-indol-2-ylidenamin,
- 4-Methoxy-1,3-dihydro-indol-2-ylidenamin,
- 2-Imino-2,3-dihydro-1*H*-indol-4-ol,
- 5-Chlor-7-methoxy-1,3-dihydro-indol-2-ylidenamin,
- 5,6-Dimethoxy-1,3-dihydro-indol-2-ylidenamin,
- 5,6-Dihydroxy-1,3-dihydro-indol-2-ylidenamin,
- 1,3-Dihydro-indol-2-ylidenamin
und den Additionssalzen dieser Verbindungen mit einer Säure.

3. Zusammensetzung nach einem der vorhergehenden Ansprüche, dadurch gekennzeichnet, daß die Additionssalze der Verbindungen der Formel (I) mit einer Säure unter Hydrochloriden, Hydrobromiden, Sulfaten, Tartraten und Acetaten ausgewählt sind.

4. Zusammensetzung nach einem der vorhergehenden Ansprüche, dadurch gekennzeichnet, daß die Verbindung oder die Verbindungen der Formel (I) 0,0005 bis 12 Gew.-% des Gesamtgewichts der Färbemittelzusammensetzung ausmachen.

5. Zusammensetzung nach Anspruch 4, dadurch gekennzeichnet, daß die Verbindung oder die Verbindungen der Formel (I) 0,005 bis 6 Gew.-% des Gesamtgewichts der Färbemittelzusammensetzung ausmachen.

6. Zusammensetzung nach einem der vorhergehenden Ansprüche, dadurch gekennzeichnet, daß die Oxidationsbase oder die Oxidationsbasen unter p-Phenylendiaminen, Bisphenylalkylendiaminen, p-Aminophenolen, o-Aminophenolen, heterocyclischen Basen und den Additionssalzen dieser Verbindungen mit einer Säure ausgewählt sind.

7. Zusammensetzung nach einem der vorhergehenden Ansprüche, dadurch gekennzeichnet, daß die Oxidationsbase oder die Oxidationsbasen etwa 0,0005 bis 12 Gew.-% des Gesamtgewichts der Färbemittelzusammensetzung ausmachen.

8. Zusammensetzung nach Anspruch 7, dadurch gekennzeichnet, daß die Oxidationsbase oder die Oxidationsbasen etwa 0,005 bis 6 Gew.-% des Gesamtgewichts der Färbemittelzusammensetzung ausmachen.

9. Zusammensetzung nach einem der vorhergehenden Ansprüche, dadurch gekennzeichnet, daß sie ferner einen oder mehrere zusätzliche Kuppler, die von den Verbindungen der Formel (I) verschieden sind, und/oder einen oder mehrere Direktfarbstoffe enthält.

10. Zusammensetzung nach einem der vorhergehenden Ansprüche, dadurch gekennzeichnet, daß das zum Färben geeignete Medium (oder der Träger) aus Wasser oder einem Gemisch von Wasser und mindestens einem organischen Lösemittel besteht, das unter niederen Alkanolen mit 1 bis 4 Kohlenstoffatomen, Glycerin, Glykolen und Glykolethern, aromatischen Alkoholen, analogen Produkten und deren Gemischen ausgewählt ist.

11. Zusammensetzung nach einem der vorhergehenden Ansprüche, dadurch gekennzeichnet, daß sie einen pH-Wert im Bereich von 3 bis 12 aufweist.

12. Zusammensetzung nach einem der vorhergehenden Ansprüche, dadurch gekennzeichnet, daß sie als Flüssigkeit, Creme, Gel oder in beliebigen weiteren Formen vorliegt, die zur Durchführung einer Färbung von Keratinfasern und insbesondere des menschlichen Haares geeignet sind.

13. Verwendung der in einem der Ansprüche 1 bis 3 definierten Verbindungen der Formel (I) als Kuppler in Zusammensetzungen zum oxidativen Färben von Keratinfasern und insbesondere menschlichen Keratinfasern, wie dem Haar, in Kombination mit mindestens einer Oxidationsbase.

14. Verfahren zum oxidativen Färben von Keratinfasern und insbesondere menschlichen Keratinfasern, wie dem Haar, dadurch gekennzeichnet, daß auf die Fasern mindestens eine in einem der Ansprüche 1 bis 12 definierte Färbemittelzusammensetzung aufgebracht wird, wobei die Farbe bei saurem, neutralem oder alkalischem pH-Wert mit einem Oxidationsmittel entwickelt wird, das unmittelbar bei der Anwendung zu der Färbemittelzusammensetzung gegeben wird oder das in einer oxidierenden Zusammensetzung vorliegt, die gleichzeitig oder getrennt davon anschließend aufgebracht wird.

15. Verfahren nach Anspruch 14, dadurch gekennzeichnet, daß das Oxidationsmittel unter Wasserstoffperoxid, Harnstoffperoxid, Alkalimetallbromaten und Salzen von Persäuren, wie Perboraten und Persulfaten, ausgewählt ist.

16. Vorrichtung mit mehreren Abteilungen oder 'Kit' zum Färben mit mehreren Abteilungen, wobei eine erste Abteilung eine in einem der Ansprüche 1 bis 12 definierte Färbemittelzusammensetzung und eine zweite Abteilung eine oxidierende Zusammensetzung enthält.

## Claims

1. Composition for dyeing keratin fibres, and in particular human keratin fibres such as the hair, characterized in that it comprises, in a medium which is suitable for dyeing:
- as coupler, at least one 2-iminoindoline derivative of formula (I), and/or at least one of the addition salts thereof with an acid, and/or at least one of the possible tautomeric forms thereof: 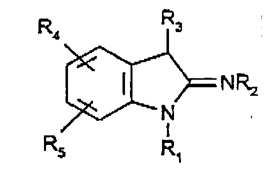 in which:
- R₁ represents a hydrogen atom, a C₁-C₄ alkyl, C₁-C₄ monohydroxyalkyl, C₂-C₄ polyhydroxyalkyl or (C₁-C₄)alkoxycarbonyl radical or a C₂-C₄ acyl radical;
- R₂ represents a hydrogen atom or a C₁-C₄ alkyl, phenyl or arylsulphonyl radical;
- R₃ represents a hydrogen atom or a C₁-C₄ alkyl radical which may be substituted with one or more hydroxyl, C₁-C₄ alkoxy, amino, mono(C₁-C₄)alkylamino, di(C₁-C₄)alkylamino, cyano or aryl groups;
- R₄ and R₅, which may be identical or different, denote a hydrogen atom or a hydroxyl, C₁-C₄ alkyl, C₁-C₄ alkoxy, amino, mono (C₁-C₄)alkylamino di (C₁-C₄)alkylamino or C₇-C₁₁ aralkyl radical, a halogen atom or a nitro group;
- and at least one oxidation base.

2. Composition according to Claim 1, characterized in that the compounds of formula (I) are chosen from:
- 4-benzyloxy-1,3-dihydroindol-2-ylideneamine,
- 4-methoxy-1,3-dihydroindol-2-ylideneamine,
- 2-imino-2,3-dihydro-1H-indol-4-ol,
- 5-chloro-7-methoxy-1,3-dihydroindol-2-ylideneamine,
- 5,6-dimethoxy-1,3-dihydroindol-2-ylideneamine,
- 5,6-dihydroxy-1,3-dihydroindol-2-ylideneamine,
- 1,3-dihydroindol-2-ylideneamine, and the addition salts thereof with an acid.

3. Composition according to either of the preceding claims, characterized in that the addition salts with an acid of the compounds of formula (I) are chosen from the hydrochlorides, hydrobromides, sulphates, tartrates and acetates.

4. composition according to any one of the preceding claims, characterized in that the compound(s) of formula (I) represent from 0.0005 to 12% by weight relative to the total weight of the dye composition.

5. Composition according to Claim 4, characterized in that the compound(s) of formula (I) represent from 0.005 to 6% by weight relative to the total weight of the dye composition.

6. Composition according to any one of the preceding claims, characterized in that the oxidation base(s) are chosen from para-phenylenediamines, bis(phenyl)alkylenediamines, para-aminophenols, ortho-aminophenols and heterocyclic bases, and the addition salts thereof with an acid.

7. Composition according to any one of the preceding claims, characterized in that the oxidation base(s) represent from 0.0005 to 12% by weight approximately relative to the total weight of the dye composition.

8. Composition according to Claim 7, characterized in that the oxidation base(s) represent from 0.005 to 6% by weight approximately relative to the total weight of the dye composition.

9. Composition according to any one of the preceding claims, characterized in that it also contains one or more additional couplers other than the compounds of formula (I) and/or one or more direct dyes.

10. Composition according to any one of the preceding claims, characterized in that the medium which is suitable for dyeing (or the support) consists of water or of a mixture of water and at least one organic solvent chosen from C₁-C₄ lower alkanols, glycerol, glycols and glycol ethers, aromatic alcohols, similar products and mixtures thereof.

11. Composition according to any one of the preceding claims, characterized in that it has a pH of between 3 and 12.

12. Composition according to any one of the preceding claims, characterized in that it is in the form of liquids, creams or gels or any other form which is suitable for dyeing keratin fibres, and in particular human hair.

13. Use of the compounds of formula (I) as defined in any one of Claims 1 to 3, as couplers in compositions for the oxidation dyeing of keratin fibres, and in particular human keratin fibres such as the hair, in combination with at least one oxidation base.

14. Process for the oxidation dyeing of keratin fibres, and in particular human keratin fibres such as the hair, characterized in that at least one dye composition as defined in any one of Claims 1 to 12 is applied to these fibres, the colour being developed at acidic, neutral or alkaline pH using an oxidizing agent which is added only at the time of use to the dye composition or which is present in an oxidizing composition that is applied simultaneously or sequentially in a separate manner.

15. Process according to Claim 14, characterized in that the oxidizing agent is chosen from hydrogen peroxide, urea peroxide, alkali metal bromates, and persalts such as perborates and persulphates.

16. Multi-compartment device or multi-compartment dyeing "kit", a first compartment of which contains a dye composition as defined in any one of Claims 1 to 12 and a second compartment of which contains an oxidizing composition
